# EUROPÄISCHE PATENTSCHRIFT

(11) **EP 0 362 339 B1**
(45) Veröffentlichungstag und Bekanntmachung des Hinweises auf die Patenterteilung: **31.05.1995**
(21) Anmeldenummer: 89903662.8
(22) Anmeldetag: 28.03.1989
(51) Int. Cl.: G01N 33/544, C12N 11/02, G01N 33/553, C12Q 1/68, G01N 33/549, C07K 17/02, A61K 9/00

(54) **Verwendung einer mit immobilisierten Molekülen bzw. Substanzen versehenen Membrane**
Use of a support with immobilized molecules or substances thereon
Utilization d'un support comprenant des molécules ou des substances immobilisées

(30) Priorität: 28.03.1988 US 174127
(43) Veröffentlichungstag der Anmeldung: 11.04.1990
(73) Patentinhaber: Sleytr, Uwe B., Dipl.-Ing., Dr., A-1170 Wien (AT); Sara, Margit, Dipl.-Ing., A-1200 Wien (AT)
(72) Erfinder: Sleytr, Uwe B., Dipl.-Ing., Dr., A-1170 Wien (AT); Sara, Margit, Dipl.-Ing., A-1200 Wien (AT)
(74) Vertreter: Itze, Peter, Dipl.-Ing.
(86) Internationale Anmeldenummer: AT8900031
(87) Internationale Veröffentlichungsnummer: WO8909406

(56) Entgegenhaltungen:
- EP-A- 154 620
- EP-A- 166 233
- EP-A- 173 500
- EP-A- 184 710
- EP-A- 189 019
- US-A- 3 979 184

## Beschreibung

Die Erfindung bezieht sich auf eine Verwendung einer mit immobilisierten Molekülen bzw. Substanzen versehenen Membran.

Bisher waren für die Immobilisierung von Makromolekülen Polymere mit schwammartiger Struktur (Gele) von verschiedenem chemischen Aufbau (z.B. Agarose, modifiziertes Polyacrylamid) verwendet. Aufgrund der zufälligen Anordnung der Polymerketten mit unterschiedlichem Molekulargewicht in dem Gelkörper weisen vorhandene funktionelle Gruppen weder eine definierte Position, noch eine definierte Orientierung auf. Die Verteilung von funktionellen Gruppen an der Oberfläche und im Inneren der Gelmatrix ist daher "zufällig". Werden diese Gruppen für eine Immobilisierung von Makromolekülen aktiviert, so wird auch die Anordnung der Fremdmoleküle in "zufälliger" Verteilung erfolgen.

Für die sogenannte Teststreifentechnologie (z.B. Glukose-Teststicks zur Bestimmung des Blut- oder Harnzuckergehaltes) werden Enzyme in gelöster Form in Qualitätspapiere eingesaugt und getrocknet. Wie bei dem zuvor beschriebenen Gel liegen auch hier die Fremdmoleküle in statistischer Verteilung in der relativ groben Fasermatrix des Papieres vor. Ein weiterer Nachteil ist, daß die Enzyme nicht kovalent gebunden sind, sodaß es bei Untersuchungen im wässrigen Milieu zu Enzymverlusten kommen kann. Dadurch ist eine Quantifizierbarkeit des Analyten nicht mehr garantiert.

Um einerseits eine kovalente Bindung von Fremdmolekülen zu sichern, anderseits aber dünne Trägerfilme zu haben, wurden Proteinfilme aus Serum Albumin oder Kollagen erzeugt. Durch Einbringen von Glutaraldehyd gelang es, den Proteinfilm zu vernetzen, und gleichzeitig Fremdmoleküle kovalent zu binden. Auch mit dieser Methode gelang es aber nicht, Fremdmoleküle in definierter Position und Orientierung in monomolekularer Schicht kovalent an einen Trägerfilm zu binden.

Aus der EP-A2-0184710 geht die Herstellung von magnetischen Microspheres hervor, an deren Oberfläche funktionelle Gruppen, die eine kovalente Bindung von biologisch aktiven Liganden erlauben, vorgesehen sind. Die beschriebenen Microspheres sind an ihrer Oberfläche mit Albumin oder anderen löslichen Proteinen beschichtet, die nach der Koagulation bzw. dem Vernetzen der einzelnen Proteinmoleküle durch bifunktionelle Crosslinker als statistisches Netzwerk vorliegen. Die Bindung von biologisch aktiven Liganden benötigt die Einbringung von funktionellen Gruppen, da die dem Protein eigenen funktionellen Gruppen vorwiegend im Inneren der Matrix liegen. Außerdem ist auf Grund des statistischen Aufbaues keine geordnete Verteilung von aktiven Liganden möglich, da ein statistisches Netzwerk keine gezielt wiederkehrende Oberflächenstruktur aufweist.

Es sind bereits als Träger dienende Membranen bekannt, welche sich entlang ebener, gekrümmter, zylindrischer oder vesikulärer Flächen erstrecken und aus wenigstens einer Schicht identischer, Protein enthaltender Moleküle bestehen, die in Form eines Kristallgitters mit einer Gitterkonstante von 1 bis 50 nm angeordnet sind. Dadurch wird erreicht, daß die immobilisierten Moleküle stets in räumlich definierter Anordnung an den mit den Substraten in Verbindung stehenden Oberflächen angeordnet sind, so daß eine möglichst dichte Belegung der freien Oberflächen mit den zu bindenden Molekülen ermöglicht ist (s. EP-A-0154 620). Die reaktiven Gruppen der Proteine nehmen nämlich nicht nur in der Polypeptidkette eine genau festgelegte Position ein, sondern haben auch nach Faltung des Proteins im Kristallgitter eine exakt festgelegte Lage und Orientierung. Dies gilt in gleicher Weise auch für an der Membran eventuell noch vorhandene Kohlenhydratanteile und deren Hydroxylgruppen.Vorteilhafterweise kann eine Membran eingesetzt werden, die Poren mit einem Durchmesser von 0,5 bis 40 nm aufweist. Durch das Vorhandensein der Poren kann eine Behandlung des Substrats auch dadurch erreicht werden, daß dieses langsam durch die Membran hindurchgeführt wird, wobei dann während des Durchführens bzw. Entlangführens die gewünschte Umsetzung bzw. Reaktion durch die an der Membran immobilisierten Moleküle erfolgt. Weiters wird damit erreicht, daß die Membran beidseitig mit den Molekülen bzw. Substanzen beladen sein kann, da aufgrund der Poren das zu behadelnde Substrat an beide Seiten der Membran gelangen und dann reagieren kann.

Für die Veränderung der Oberflächeneigenschaften der verwendeten Membran können auf dieser nieder- oder hochmolekulare Fremdmoleküle, z.B. Protein- und/oder Peptidmoleküle, immobilisiert sein. Dadurch wird unter anderem eine Oberflächenvergrößerung erreicht, wodurch für Immobilisierung weiterer Moleküle ein zusätzlicher Raum geschaffen wird, der aufgrund der definierten Anordnung der Protein- und/oder Peptidmoleküle ebenfalls genau definierte Raumstruktur aufweist. Weiters kann dadurch erreicht werden, daß die Oberfläche entweder hydrophil bzw. hydrophob wird. Durch solche Veränderungen der Oberflächeneigenschaften wird unter anderem auch erzielt, daß die Membranen eine geringere unspezifische Adsorption aufweisen. Gleiche Eigenschaften können auch dadurch erzielt werden, daß auf der Membran Glycoprotein- und/oder Glycopeptidmoleküle immobilisiert werden. Ebenso können auf der Membran Polysaccharide und/oder Oligosaccharide und/oder Zucker immobilisiert werden, wobei all die angeführten Moleküle je nach dem späteren Verwendungszweck eingesetzt werden können.

Es können jedoch zur Erzielung dieser Eigenschaften auch Lipidmoleküle immobilisiert sein, wobei diese Moleküle zusätzlich noch für die Aufbringung von monomolekularen Lipidschichten, z.B. Langmuir Blodget-Filmen, dienen können, oder aber als Träger für andere Polymerschichten, insbesondere hydrophobe Membranen, dienen können. Für spezielle Zwecke können auch auf der verwendeten Membran Lipopolysaccharide immobilisiert sein, welche gleichfalls die angeführten Effekte erbringen können.

Für einen Einsatz bei enzymatischen Umsetzungen können auf der verwendeten Membran Enzyme und/oder Coenzyme immobilisiert sein. Soll die Membran für biotechnologische Zwecke verwendet werden, dann kann auf der verwendeten Membran eine Substanz der Gruppe, enthaltend Antigene, Antikörper, Lektine, Biotin, Avidin, Protein A und Haptene, immobilisiert sein. Für gewisse Zwecke können auch Nucleinsäuren immobilisiert sein, welche sich unter anderem auch für Hybridisierungstests und Sondenmoleküle in der Gentechnologie eignen.

Wenn auf der verwendeten Membran Farbstoffe, insbesondere Fluoreszenzfarbstoffe, immobilisiert sind, dann kann die Membran als Testmembran dienen, z.B. als Indikatormembran bzw. als Membran, welche insbesondere für optoelektronische Auswertung (optische Sensoren) geeignet ist. Es können dabei für die Auswertung Membrangruppen verwendet werden, von welchen jede Membran einen unterschiedlichen Farbstoff aufweist, so daß gleichzeitig unterschiedliche Reaktionen gemessen werden können.

Es können auf der verwendeten Membran, gegebenenfalls leitende, Materialien, wie Metalle und/oder Metallverbindungen und/oder Kohlenstoff und/oder Siliciumoxide und/oder Kunststoffe immobilisiert bzw. abgelagert sein. Dadurch ist die Möglichkeit gegeben, die Membranen für Sensoren einzusetzen, u.zw. beispielsweise als biologische Feldeffekttransistoren oder chemische Feldeffekttransistoren, da die geschaffenen Strukturen ionenselektiv sind und damit als sogenannte ionenselektive Feldeffekttransistoren (ISFET) eingesetzt werden können. Für die Steuerung der Struktur bzw. die physikalischen Eigenschaften und Herstellung von Legierungen auf der verwendeten Membran können Mischungen der Materialien bzw. getrennte Schichten mehrerer Materialien abgelagert sein.

Um eine gesteuerte Elektronenübertragung bzw. Ionenableitung aus dem Medium zu erzielen, können auf der verwendeten Membran Mediator- bzw. Transmittermoleküle immobilisiert sein.

Erfindungsgemäß können diese Membranen ganz gezielt eingesetzt werden, u. zw., um bei enzymgesteuerten Prozessen ein leichtes Rückgewinnen der eingesetzten Enzyme zu erzielen, wird eine Membran mit darauf immobilisierten Enzymen und/oder Coenzymen als Enzymmembran verwendet werden. Es wird dann das Substrat mit der Enzymmembran in Kontakt gebracht bzw., wenn die Membran vesikuläre Form hat, dieses Vesikel in dem Substrat suspendiert, wonach dann nach Beendigung der Reaktion das Substrat von der Membran getrennt wird bzw. die Vesikel, z.B. durch Filtration, abgeschieden werden. Eine Membran mit darauf immobilisierten Antigenen, Antikörpern, Lektinen, Biotin, Avidin, Protein A oder Haptenen wird als ELISA-Membran verwendet. Für die Durchführung von Diagnosereaktion wird eine Membran mit darauf immobilisierten Enzymen, bzw. Coenzymen, Antigenen, Antikörpern, Biotin, Avidin, Lektinen, Protein A, Haptenen, Nucleinsäuren oder Mediator- bzw. Transmittermolekülen als Diagnosereagens verwendet werden. Insbesondere bei den Enzymen ist eine Biolumineszenzreaktion interessant, weil dadurch eine lichtoptische Auswertung von Reaktionen ermöglicht ist. Eine Membran mit darauf immobilisierten Enzymen bzw. Coenzymen, Antigenen, Antikörpern, Biotin, Avidin, Lektinen, Protein A, Haptenen, Nucleinsäuren oder Mediator- bzw. Transmittermolekülen, ist auch als Sensormembran verwendbar, welche einen direkten Einsatz für die Prüfung von Substanzen auf vorhandene Komponenten ermöglicht.

Für bestimmten Formen der Signalableitungen, insbesondere für Redoxsysteme, können die an der verwendeten Membran immobilisierten Substanzen und die Membran mit einer leitenden Schicht, z.B. Metall oder leitendem Kunststoff, versehen sein. In besonders einfacher Weise kann die leitende Schicht durch Sputtern aufgebracht sein. Für bestimmte Materialien kann es jedoch angezeigt sein, daß die leitende Schicht durch Aufdampfen aufgebracht wird. Schließlich kann die Kunststoffschicht durch Plasmapolymerisation aufgebracht werden.

Für eine gesteuerte Freisetzung der abgelagerten bzw. immobilisierten Substanzen, insbesondere bei.... pharmazeutischen Wirkstoffen, können diese Substanzen von der Membran eingeschlossen sein.

Die Erfindung wird nachstehend noch anhand von Beispielen näher erläutert.

### Beispiel 1:

### Immobilisierung von Poly-L-Lysin an kristallinen Proteinmembranen (S-Schichten)

Vesikuläre Strukturen, die aus kristallinen Proteinmembranen von Clostridium thermohydrosulfuricum L111-69 bestehen, werden zur Stabilisierung mit Glutaraldehyd vernetzt. Dazu werden 0.5 g feuchtes Pellet der vesikulären Strukturen (erholten noch Zentrifugation bei 20,000 × g) in 50 ml 0.1 M Natrium-Cacodylat-Puffer, pH 7.2, suspendiert und nach Zugabe von 0.5 ml Glutaraldehyd (50 %) 20 Minuten bei 20° C inkubiert. Nach Stoppen der Reaktion durch Zugabe von Äthanolamin wird die Suspension bei 20,000 × g zentrifugiert, dreimal mit Aqua dest. gewaschen und neuerlich pelletiert. Zur chemischen Aktivierung der Carboxylgruppen werden 0.2 g des erhaltenen Pellets in 8 ml Aqua dest. suspendiert, 60 mg 1-Ethyl-3,3′ dimethyl(aminopropyl)carbodiimid (EDC) zugefügt, der pH-Wert der Suspension durch Zugabe von 0.1 N NaOH oder 0.1 N HCl auf 4.63 eingestellt und dieser während der 80 Minuten dauernden Reaktion konstant gehalten. Nach Zentrifugation bei 20,000 × g wird das Pellet mit eiskaltem Aqua dest. gewaschen und nach neuerlichem Zentrifugieren bei 20,000 × g in einer Lösung aus Poly-L-Lysin (MG 30,000 ; 2 mg/ml Aqua dest.) suspendiert. Nach 4-stündiger Inkubation bei 20° C wird zur Entfernung von nicht kovalent gebundenem Poly-L-Lysin zentrifugiert, mit Aqua dest., und in der Folge mit 0.1 M Kaliumphosphat Puffer (pH 7.0) gewaschen.

Durch Immobilisierung von Poly-L-Lysin können aufgrund der freien Aminogruppen der im Polymer vorhandenen Lysinreste die Oberflächeneigenschaften der vesikulären Strukturen (Proteinfragmente) gezielt verändert und eine positive Nettoladung erzeugt werden. Zudem können die vorhandenen Aminogruppen für eine Immobilisierung von weiteren Fremdmolakülen aktiviert werden.

### Beispiel 2:

### Immobilisierung von Phosphatidylethonolamin

Vesikuläre Strukturen werden, wie oben beschrieben, zur chemischen Stabilisierung mit Glutaraldehyd vernetzt und an der kristallinen Matrix vorhandene Carboxylgruppen mit EDC aktiviert. Nach einer Aktivierungszeit von 80 Minuten wird die Suspension bei 20,000 × g zentrifugiert, die Pellets mit Dioxan bei einer Temperatur von 4° C gewaschen und neuerlich sedimentiert. In der Folge werden die Pellets in einer Lösung aus Phosphatidylethanolamin (PE; 3 mg/ml Dioxan) gelöst und 20 h bei 20° C inkubiert. Zur Entfernung von nicht kovalent gebundenem PE werden die vesikulären Strukturen nach dem Zentrifugieren fünfmal mit Dioxan und anschließend mit einer Mischung aus Chloroform-Methanol (50/50) gewaschen. Durch Reaktion der freien Aminogruppen von PE mit den Carboxylgruppen des S-Schicht-Proteins gelingt es, PE so an die kristalline Matrix zu binden, daß der hydrophile Teil zu der Membranoberfläche gerichtet ist, während die hydrophoben Reste nach außen exponiert bleiben. Die so erzeugte hydrophobe Oberfläche dient zur Anlagerung von Monoschichten aus Tensiden (z.B. Arachinsäure), die nun ihrerseits mit ihrem hydrophoben Teil binden, während der hydrophile Teil nach außen exponiert ist. Nun ist eine weitere Anlagerung einer Monoschicht von Arachinsäure möglich, wobei nun die Moleküle mit ihrer hydrophilen Seite binden. Nach diesem Prinzip können mehrere Monoschichten von Tensidmolekülen an der Oberfläche einer kristallin geordneten vesikulären Struktur angelagert werden.

### Beispiel 3:

### Immobilisierung von Glucoseoxidase an kristallinen vesikulären Strukturen

Zur Immobilisierung von Glucoseoxidase werden vesikuläre Strukturen, wie oben beschrieben, mit Glutaraldehyd vernetzt, und in der Folge im Kristallgitter vorhandene Carboxylgruppen mit EDC aktiviert. Nach dem Waschen mit Eiswasser und neuerlichem Zentrifugieren bei 20,000 × g wird das aktivierte Pellet aus vesikulären Strukturen in einer Lösung aus Glucoseoxidase (3 mg/ml Aqua dest.) suspendiert und 6 Stunden bei 20° C inkubiert. In der Folge wird die Suspension zentrifugiert und, wie in Beispiel 1 beschrieben, zur Entfernung von nicht kovalent gebundenem Material gewaschen. Unter Anwendung dieser Methode ist es möglich, 1 mg Glucoseoxidase pro mg S-Schicht-Protein kovalent zu binden. Das bedeutet, daß pro S-Schicht-Untereinheit ein Glucoseoxidase-Molekül immobilisiert werden kann, was der dichtest möglichen Packung eines Fremdmoleküles an einer kristallinen Matrix entspricht. Immobilisierte Glucoseoxidase zeigte im Vergleich zu dem nativen Enzym eine Aktivität von 50 %.

### Beispiel 4:

### Belegen von kristallinen S-Schichten mit einer Metallschicht

Kristalline geordnete Membranen (S-Schichten) werden in Aqua dest. suspendiert und unter einem Druck von 2 bar in einer 10 ml Ultrafiltrationszelle auf einen mikroporösen Träger (Mikrofiltrationsmembran aus Polycarbonat der Firma Nuclepore; Porendurchmesser 0.1 »m) angeschwemmt. Die einzelnen Membranfragmente werden in der Folge mit Glutaraldehyd (0.5 % in 0.1 M Natrium Cacodylat Puffer, pH 7.0) vernetzt. Nach einer Inkubationszeit von 20 Minuten wird durch Waschen mit Aqua dest. überschüssiges Reagens entfernt, die Mikrofiltrationsmembran mit den abgelagerten Protein-Fragmenten der Ultrafiltrationszelle entnommen und luftgetrocknet. Anschließend wird die Struktur in eine Sputter Coater Anlage (Polaron Instruments) eingelegt und bis zu einem Vakuum von 5.10⁻² Torr evakuiert. Nun wird durch Anlegen einer Spannung von 2.000 V bei einem Stromfluß von etwa 20 mA Gold auf die Oberfläche der kristallinen Membranfragmente gesputtert, wodurch eine zusammenhängende Metallschicht mit einer Stärke von etwa 40 nm erzeugt wird. Nach dem Entnehmen aus der Sputter Coater Anlage wird die Struktur vorsichtig in ein mit Chloroform gefülltes Becherglas transferiert und so eingelegt, daß die Polycarbonat-Trägermembran mit der Flüssigkeit direkt in Kontakt kommt, wodurch das vorhandene Polymer aufgelöst wird. An der Flüssigkeitsoberfläche verbleibt nun eine extrem dünne Struktur, die aus der Goldschicht und den darunterliegenden S-Schicht-Fragmenten besteht. Diese Struktur kann nun durch vorsichtiges Abheben auf beliebige Oberflächen aufgebracht werden. Durch Aufsputtern von Gold auf kristallin geordnete Membranfragmente ist es möglich, einen direkten Kontakt zwischen Metall und dem Träger zu erzielen.

### Beispiel 5:

### Immobilisierung von Fluoreszenzfarbstoffen an kristallin geordnete Strukturen

Vesikuläre Strukturen, die aus kristallin angeordneten Proteinuntereinheiten bestehen, werden zur kovalenten Bindung des Fluoreszenzfarbstoffes 7-Hydroxy-Cumarin-3--carbonsäure (HCC), der einen pH-sensitiven Farbstoff darstellt, verwendet. 50 mg des Farbstoffes werden in Aqua dest. aufgelöst, der pH-Wert durch Zugabe von 0.1 N HCl oder 0.1 N NaOH auf 4,75 eingestellt und in der Folge 20 mg EDC zugesetzt. Nach einer Aktivierung der Carboxylgruppen von HCC während einer Reaktionszeit von 80 Minuten wird dieser Ansatz mit 0,5 g feuchtem Pellet vesikulärer Strukturen (erhalten nach Zentrifugation bei 20,000 × g) vermischt und die Suspension 2 Stunden bei 20° C inkubiert. Während dieser Zeit können die EDC-aktivierten Carboxylgruppen von HCC mit freien Aminogruppen des S-Schicht-Proteins reagieren und der Farbstoff kovalent an die kristalline Matrix gebunden werden. Zur Entfernung überschüssigen Reagens wird die Suspension in einen vorgeweichten Dialysierschlauch übergeführt und 48 h lang bei einer Temperatur von 4° C gegen Aqua dest. dialysiert. Niedermolekulare Verbindungen, wie HCC oder EDC, können während der Dialyse entfernt werden. Die Suspension wird in der Folge zentrifugiert, mit Aqua dest. und 1 N NaCl zur Entfernung von unspezifisch adsorbiertem Material gewaschen. Der an den vesikulären Strukturen immobilisierte Fluoreszenzfarbstoff reagiert auf Änderungen des pH-Wertes im entsprechenden Milieu durch eine Änderung in der Wellenlänge der ausgesandten Fluoreszenz.

### Beispiel 6:

### Immobilisierung von Glucoseoxidase an kristalline Protein-Fragments und Belegung mit einer Metallschicht

Die Immobilisierung von Glucoseoxidase erfolgt an kristallin geordnete Protein-Fragmente, die auf einem mikroporösen Träger (Mikrofiltrationsmembran aus Nylon der Firma Pall; Porendurchmesser 0.1 m) abgelagert wurden. Die Mikrofiltrationsmembran wird in eine Ultrafiltrationszelle mit einem Durchmesser von 25 mm eingelegt, und 3 ml einer Suspension, die die Protein-Fragmente enthält, werden auf die Membranoberfläche pipettiert. Nach Anlegen von einem Druck von 2 bar und Ablagerung der Protein-Fragmente an der porösen Trägermembran wird vorhandenes Protein durch Zugabe von Glutaraldehyd (0.5 % in 0.1 M Natrium-Cacodylat-Puffer, pH 7.2) kovalent vernetzt. Nach dem Waschen der Membran mit Aqua dest. wird diese aus der Ultrafiltrationszelle entnommen und in ein Becherglas, das 10 ml Aqua dest. enthält, eingelegt. Nach Zugabe von 50 mg EDC zur Aktivierung von Carboxylgruppen wird der pH-Wert während der folgenden 80 Minuten auf 4.65 konstant gehalten. Durch Eintauchen der Membranen in Eiswasser läßt sich überschüssiges Reagens entfernen. Die Membran wird in die Ultrafiltrationszelle nun so eingelegt, daß die mit Protein-Fragmenten belegte Seite nach oben gerichtet ist. In der Folge werden 2 ml einer Glucoseoxidase-Lösung (2 mg/ml) zugefügt und zur kovalenten Bindung des Enzyms 2 Stunden bei 20° C inkubiert. Nicht kovalent gebundenes Enzym kann durch Waschen mit Aqua dest. und Puffer (siehe oben) entfernt werden. Nach dem Entnehmen der Membran aus der Ultrafiltrationszelle wird diese luftgetrocknet, in eine Sputter Coater Anlage (Polaron Instruments) eingelegt und bei einem Druck von 5.10⁻² Torr mit Platin bei einer Stromstärke von 20 mA während eines Zeitraumes von 2 min besputtert. Die Metallschicht, die eine Stärke von 30 nm aufweist, ist direkt mit dem Enzym, das in dichtest möglicher Packung an eine kristalline Matrix gebunden ist, in Kontakt. Dadurch ist es möglich, daß die an dem Enzym während der enzymatischen Reaktion entstehenden Elektronen direkt von der Platinschicht abgesaugt werden.

### Beispiel 7:

### Herstellung von Diagnosemembranen unter Verwendung von Antikörpern

Als Ausgangsmaterial werden an mikroporöse Träger gebundene Protein-Fragmente verwendet. An der Oberfläche der Protein-Fragmente vorhandene Carboxylgruppen werden mit EDC aktiviert (Durchführung siehe o.a. Beispiele). Anschließend wird die aktivierte Membran in eine Ultrafiltrationszelle mit einem Durchmesser von 25 mm eingelegt und 2 ml einer Antikörper-Lösung (0.4 mg/ml) (Antikörper A, erzeugt gegen Virusprotein) auf die Membranoberfläche pipettiert und 4 Stunden bei 20° C inkubiert. Nach Abdekantieren der Antikörper-Lösung wird die Membran aus der Ultrafiltrationszelle genommen und 2 h mit 0.2 M Kalium-Phosphat-Puffer, pH 7.2, gewaschen. Die Antikörper-Moleküle wurden im Zuge der Immobilisierung in Form einer Monoschicht in dichtest möglicher Packung an die Oberfläche der kristallinen Matrix gebunden. Die mit Antikörper beladene Membran kann daher zum Nachweis von Virusprotein verwendet werden. Dazu wird die Membran mit einer Flüssigkeit, die Viren enthält, in Kontakt gebracht und 2 h bei 20° C inkubiert. In der Folge wird zur Entfernung von überschüssigem Antigen mit 0.2 M Phosphat-Puffer, pH 7.0, und mit Aqua dest. gewaschen. Zur quantitativen Auswertung der gebundenen Virusproteinmenge wird nun die Membran in eine weitere Lösung, die biotinylierten Antikörper A enthält, gelegt (0.1 mg/ml 0.1 N Natriumhydrogencarbonat) und 30 Minuten inkubiert. Der biotinylierte Antikörper bindet nun an noch freie Haptene des Virusproteins. Nach Entnehmen der Membran aus der Antikörper-Lösung wird diese wieder 20 Minuten mit 0.1 M Phosphat-Puffer, pH 8.0, gewaschen. Zur quantitativen Auswertung wird nun ein Peroxidase-Avidin-Konjugat eingesetzt. Avidin bindet spezifisch an Biotin, und demnach an den Jeweiligen biotinylierten Antikörper. Nach einer Inkubationszeit von 10 Minuten wird die Membran entnommen, wieder 20 Minuten mit Phosphat-Puffer gewaschen und schließlich 1 ml einer 0.01 % Wasserstoffperoxid-Lösung, die 1 mg des Farbstoffes o-Dianisidin enthält, auf die Membran getropft. Nach 10 Minuten wird die Reaktion durch Zugabe von 5N HCl gestoppt und der gebildete Farbstoff quantitativ durch Messung bei einer Wellenlänge von 400 nm bestimmt. Über eine entsprechend erstellte Eichkurve kann die Menge an gebundenem Virusprotein gemessen werden.
Kristallin geordnete Protein-Fragmente bieten den Vorteil, daß der Antikörper A in dichtest möglicher Packung an eine definierte Oberfläche gebunden werden kann und dadurch die Nachweisgrenze für das zu untersuchende Protein entsprechend niedriger ist.

### Beispiel 8:

### Immobilisierung von biotinylieten Proteinen an vesikuläre Strukturen

Vesikuläre Strukturen, die aus Proteinkristallen bestehen, werden entsprechend der oben angeführten Vorschrift chemisch mit Glutaraldehyd vernetzt und die an der Oberfläche vorhandenen Carboxylgruppen mit EDC aktiviert. 0.1 g des feuchten, EDC-aktivierten Pellets werden mit 2 ml einer Lösung von biotinyliertem Ovalbumin 1 mg/ml Aqua dest.) vermischt und 2 h bei 20° C inkubiert. Anschließand wird bei 20,000 × g zentrifugiert und das Pellet fünfmal mit 0.1 M Kalium-Phosphat-Puffer, pH 7.0, gewaschen. Pro Protein-Untereinheit der kristallinen Matrix konnten 2 Moleküle von biotinyliertem Ovalbumin gebunden werden, was wieder der dichtest möglichen Bindungsdichte eines Fremdmoleküles an eine kristalline Matrix entspricht. Immobilisiertes biotinyliertes Ovalbumin kann nun durch Zugabe von Avidin-Peroxidase-Konjugat und im folgenden durch Messung des entwickelten Farbstoffes durch Peroxidase-Aktivität nachgewiesen werden. Dazu werden 50 mg des feuchten Pellets aus vesikulären Strukturen mit daran immobilisiertem biotinylierten Ovalbumin in 1 ml 0.1 M Natriumhydrogencarbonat, pH 8.5, suspendiert und 200 »l 0.2 % Avidin-Peroxidase-Konjugat dazugefügt. Nach 20 Minuten Inkubation bei 20° C wird zentrifugiert, das Pellet fünfmal mit 0.2 M Kalium-Phosphat-Puffer (pH 7.0) gewaschen und 20 mg des Pellets (bestehend aus vesikularen Strukturen, biotinyliertem Ovalbumin und daran gebundenen Avidin-Peroxidase-Konjugat) mit 200 »l einer Lösung von 0.01 % Wasserstoffperoxid, die 1 mg o-Dianisidin enthält, vermischt. Der entwickelte Farbstoff wird, wie oben beschrieben, gemessen.

## Patentansprüche

1. Verwendung einer Membran, die sich entlang ebener, gekrümmter oder vesikulärer Flächen erstreckt und aus wenigstens einer Schicht identischer, Protein enthaltender, in Form eines Kristallgitters mit einer Gitterkonstante von 1 bis 50 nm angeordneter Moleküle besteht und auf welcher eine Substanz der Gruppe enthaltend Enzyme bzw. Coenzyme, Antigene, Antikörper, Biotin, Avidin, Lektine, Protein A, Haptene, Lipidmoleküle, Lipopolysaccharride, Nucleinsäuren oder Mediator- bzw. Transmittermoleküle immobilisiert sind, als Enzymmembran und/oder ELISA-Membran und/oder Diagnosereagenz und/oder Sensormembran.

2. Verwendung nach Anspruch 1, bei welcher die an der Membran immobilisierten Substanzen und die Membran mit einer leitenden Schicht. z.B. Metall oder leitendem Kunststoff, oder Siliciumoxide oder Kohlenstoff, Mediator- und Transmittermoleküle, versehen sind.

3. Verwendung nach Anspruch 2, bei welcher die leitende Schicht durch Sputtern aufgebracht ist.

4. Verwendung nach Anspruch 2, bei welcher die leitende Schicht durch Aufdampfen aufgebracht ist.

5. Verwendung nach Anspruch 2, bei welcher die leitende Kunststoffschicht durch Plasmapolymerisation aufgebracht ist.

## Claims

1. Use of a membrane which extends along planar, curved or vesicular surfaces and consists of at least one layer of identical, protein-containing molecules arranged in the form of a crystal lattice with a lattice constant of 1 to 50 nm and on which is immobilised a substance from the group comprising enzymes or coenzymes, antigens, antibodies, biotin, avidin, lectins, protein A, haptens, lipid molecules, lipopolysaccharides, nucleic acids or mediator or transmitter molecules, as an enzyme membrane and/or ELISA membrane and/or diagnostic reagent and/or sensor membrane.

2. Use according to claim 1, wherein the substances immobilised on the membrane and the membrane itself are provided with a conductive layer, e.g. metal or conductive plastics or silicon oxides or carbon, mediator and transmitter molecules.

3. Use according to claim 2, wherein the conductive layer is applied by sputtering.

4. Use according to claim 2, wherein the conductive layer is applied by vaporisation.

5. Use according to claim 2, wherein the conductive plastics layer is applied by plasma polymerisation.

## Revendications

1. Utilisation d'une membrane, qui s'étend le long de surfaces planes, incurvées ou vésiculaires, et qui est constituée par au moins une couche de molécules contenant des protéines identiques et disposées en un réseau cristallin ayant une constante de réseau comprise entre 1 et 50 nm, et sur laquelle une substance appartenant au groupe comprenant les enzymes, les coenzymes, les antigènes, les anticorps, la biotine, l'avidine, la lectine, la protéine A, les haptènes, les molécules lipidiques, les lipopolysaccharides, les acides nucléiques et les molécules médiatrices et transmettrices, est immobilisée, comme membrane enzymatique et/ou comme membrane de test ELISA et/ou comme réactif de diagnostic et/ou comme membrane de détection.

2. Utilisation selon la revendication 1, selon laquelle les substances immobilisées sur la membrane et la membrane sont pourvues d'une couche conductrice par exemple un métal ou une matière synthétique conductrice ou un oxyde de silicium ou du carbone, une molécule médiatrice et transmettrice.

3. Utilisation selon la revendication 2, selon laquelle la couche conductrice est appliquée par pulvérisation.

4. Utilisation selon la revendication 2, selon laquelle la couche conductrice est appliquée par métallisation sous vide.

5. Utilisation selon la revendication 2, selon laquelle la couche de matière synthétique conductrice est appliquée par polymérisation du plasma.
